# FASCICULE DE BREVET EUROPEEN

(11) **EP 1 345 605 B1**
(45) Date de publication et mention de la délivrance du brevet: **20.07.2005**
(21) Numéro de dépôt: 01989653.9
(22) Date de dépôt: 21.12.2001
(51) Int. Cl.: A61K 31/40, A61K 9/16, A61K 9/20

(54) **COMPOSITION PHARMACEUTIQUE SOLIDE THERMOFORMée POUR LA LIBERATION CONTROLEE DE PERINDOPRIL**
WARMGEFORMTE FESTE ARZNEIZUSAMMENSETZUNG ZUR GESTEUERTEN WIRKSTOFFABGABE VON PERINDOPRIL
THERMOFORMed SOLID PHARMACEUTICAL COMPOSITION FOR CONTROLLED RELEASE OF PERINDOPRIL

(30) Priorité: 26.12.2000 FR 0017013
(43) Date de publication de la demande: 24.09.2003
(73) Titulaire: Les Laboratoires Servier, 92415 Courbevoie Cedex (FR)
(72) Inventeur: WUTHRICH, Patrick, F-45000 Orléans (FR); ROLLAND, Hervé, F-45160 Olivet (FR); BRIAULT, Gilles, F-91670 Angerville (FR); PICHON, Gérald, F-45100 Orléans (FR); THARRAULT, François, F-45000 Orléans (FR)
(86) Numéro de dépôt international: PCT/FR2001/004133
(87) Numéro de publication internationale: WO 2002/051407

(56) Documents cités:
- EP-A- 0 544 144
- WO-A-98/56355
- GB-A- 2 352 172
- US-A- 5 965 161

## Description

La présente invention concerne une nouvelle composition pharmaceutique solide, pour la libération contrôlée de Périndopril, obtenue par thermoformage à chaud d'un mélange à base de polymère(s) appartenant à la famille des polyméthacrylates.

De nombreuses compositions pharmaceutiques destinées à la libération contrôlée de principes actifs pharmaceutiques ont été proposées et réalisées, pour leur administration par voie orale, buccale, sublinguale, oculaire, rectale, vaginale et/ou parentérale. Ces nouvelles compositions avaient essentiellement pour objectifs :
- de réduire la fréquence d'administration des médicaments,
- d'obtenir des taux relativement constants de principe actif dans le milieu ou au site biologique visé,
- d'obtenir des profils de libération en corrélation avec l'activité pharmacologique des médicaments.
Pour contrôler cette libération, le principe le plus communément employé est d'incorporer le ou les principes actifs avec des excipients, le plus souvent de nature polymérique, dans des matrices.

Quelles que soient les compositions matricielles envisagées, leur obtention se heurte à des problèmes spécifiques de fabrication :
- procédé de fabrication complexe et en plusieurs étapes,
- stabilité du principe actif au cours du procédé de fabrication et vis-à-vis des excipients utilisés,
- modulation de la vitesse de libération, du ou des principes actifs, délicate, souvent variable dans le temps et dépendante par exemple de la granulométrie des lots de polymères avec les procédés de compression,
- procédé de fabrication permettant l'obtention d'une forme pharmaceutique essentiellement adaptée à une seule voie d'administration,
- reproductibilité des lots du fait de la multiplication des étapes.

Le Perindopril, composé de formule : 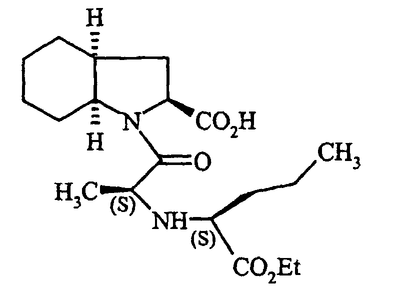 possède des propriétés pharmacologiques intéressantes. Il exerce notamment une activité inhibitrice sur certaines enzymes, comme les carboxypolypeptidases, les enképhalinases ou la kininase II. Il inhibe notamment la transformation du décapeptide angiotensine I en l'octapeptide angiotensine II, responsable dans certains cas de l'hypertension artérielle, en agissant sur l'enzyme de conversion.

L'emploi en thérapeutique du Périndopril et de ses sels pharmaceutiquement acceptables permet de réduire ou même supprimer l'activité de ces enzymes responsables de la maladie hypertensive ou de l'insuffisance cardiaque. L'action sur la kininase II a pour résultat l'augmentation de la bradykinine circulante et également la baisse de la tension artérielle par cette voie.

Actuellement, le sel de *tert*-butylamine du Périndopril est administré par voie orale sous la forme d'une composition pharmaceutique à libération immédiate. Il était donc particulièrement intéressant de proposer de nouvelles compositions pharmaceutiques permettant la libération contrôlée de ce principe actif.

L'objet de la présente invention est une alternative permettant de surmonter les difficultés d'ordre général précédemment exposées pour l'obtention de compositions pharmaceutiques solides permettant la libération contrôlée de Périndopril et de ses sels pharmaceutiquement acceptables par l'utilisation des techniques de thermoformage. Elle permet notamment de réduire le nombre d'étape pour la production de formes galéniques finalisées, limitant ainsi les problèmes de reproductibilité et le coût économique, et assurant aussi un gain de temps et d'espace au sein d'une chaîne de production.

Plus particulièrement, l'invention vise une nouvelle application des polyméthacrylates à la réalisation desdites compositions pharmaceutiques solides sans addition de plastifiant et sans addition d'agents modulant la libération du ou des principes actifs; L'invention, telle que mise en oeuvre par la Demanderesse permet ainsi de restreindre le nombre de produit intervenant au sein d'une formulation galénique, limitant ainsi les problèmes de stockage et d'approvisionnement, ainsi que les problèmes liés à la gestion de l'environnement.

Le thermoformage à chaud concerne notamment les techniques de l'extrusion, de la co-extrusion, de l'injection et de la co-injection. Ces différentes techniques sont bien connues dans le domaine de la plasturgie et ont été largement utilisées dans le secteur de l'automobile ou de l'emballage.

De part leurs caractéristiques, et les propriétés physicochimiques des polymères utilisables pour le thermoformage, ces techniques, et notamment l'extrusion simple, sont de plus en plus appliquées au domaine de la mise en forme des principes actifs.

Différents brevets décrivent ainsi des compositions pharmaceutiques à libération contrôlée qui sont obtenues par extrusion d'un mélange comportant au moins, un principe actif, un ou plusieurs polymères extrudables et pharmaceutiquement acceptables, un plastifiant et/ou un retardant, ce dernier composé permettant de moduler la libération du principe actif.

C'est le cas plus particulièrement de la demande de brevet WO 96/14058 qui revendique une composition pharmaceutique incluant notamment comme agent thérapeutique un opioïde qui est dispersé dans une matrice réalisée par extrusion. La matrice à extruder comprend ainsi un principe actif, un matériel hydrophobe qui peut être fondu, tel qu'un alkyle cellulose ou un polymère acrylique ou méthacrylique, et un matériel hydrophobe tel qu'un acide gras ou un alcool gras. Ce dernier composé joue le rôle de retardant et permet de ralentir et contrôler la libération dudit principe actif. Afin de diminuer la température d'extrusion, un plastifiant est ajouté au mélange.

Le brevet US 5,102,668 décrit une composition pharmaceutique à libération contrôlée, indépendante du pH, ladite composition étant obtenue par extrusion humide de polymères tels que les polyméthacrylates, lesdits polymères étant hydrophiles à faible pH et hydrophobes à fort pH. Le polyméthacrylate utilisé préférentiellement est de l'Eudragit® E100. Les extrudats ainsi obtenus doivent ensuite subir une étape de sphéronisation, puis de façon avantageuse , ils sont recouverts d'un film de polymère constitué d'Eudragit® NE 30 D. L'association entre le polymère constituant l'extrudat et le polymère constituant le film de revêtement permet de résoudre le problème technique particulier de cette invention qui est le contrôle de la libération du principe actif en fonction du pH du milieu de dissolution.

Parmi l'art antérieur, on peut également citer le brevet DE 41 38 513 qui présente un procédé de préparation d'une composition pharmaceutique à libération contrôlée, par extrusion en continu d'un mélange comprenant au moins un principe actif, un polyméthacrylate, et un polymère de N-vinylpyrrolidone et/ou d'hydroxyalkyl (méthyl)cellulose. Ces derniers composés sont utilisés en tant que plastifiant et jouent un rôle dans la régulation de la libération contrôlée du principe actif.

Dans l'article *Pharm. Res*. 1996, 13 (5), 804-808, il est également décrit l'extrusion à chaud d'Eudragit® E100 additionné de plastifiant, au moins 12 % de triéthylcitrate, pour l'obtention de films permettant la libération contrôlée de principes actifs.

De même, les revues *J. Cont. Rel.* 1995, 36, 243-250 et *Drug Dev. Ind. Pharm.* 1994, 20, 1323-1339 rapportent l'utilisation de l'Eudragit® RS PM additionné de plastifiant (triacetine) pour l'obtention de granules par extrusion à chaud. La cinétique de libération du principe actif est rapide et les granules ne libèrent pas la totalité du principe actif. Les températures d'extrusion se situent entre 130°C et 140°C.

Ces différents documents décrivent ainsi l'application de la technique de l'extrusion simple pour l'obtention de nouvelles compositions pharmaceutiques. Les techniques de l'injection et de la co-injection ont été beaucoup moins étudiées et elles concernent principalement des compositions pharmaceutiques solides dont la matrice est à base de dérivés cellulosés, d'amidon ou de polyéthylène glycol.

Enfin, concernant la technique de la co-extrusion, la demande de brevet FR 2 766 088 décrit un procédé pour la production d'un article à partir duquel peuvent être fabriqués des dispositifs à libération contrôlée, ledit procédé consistant à effectuer une co-extrusion de polymère et de principe actif, le polymère utilisé étant de préférence un composé organosilicié capable de se réticuler en la présence ou en l'absence d'agent de réticulation.

La présente invention permet, d'une façon simple et économique, d'obtenir directement une composition pharmaceutique solide, à libération contrôlée, par simple mélange de Périndopril ou d'un de ses sels pharmaceutiquement acceptables et de polymère(s) ayant des propriétés plastiques et étant pharmaceutiquement acceptables, sans ajout de plastifiant ou de retardant, ledit mélange étant thermoformé. Le contrôle de la libération du principe actif contenu dans ladite composition est obtenu uniquement grâce à un choix judicieux du ou des polymères plastiques utilisés, et de leur quantité relative par rapport à celle du principe actif. Les compositions pharmaceutiques selon l'invention, outre le fait qu'elles soient nouvelles, permettent d'obtenir des formes galéniques aisément adaptables au Périndopril et à ses sels pharmaceutiquement acceptables et à son meilleur mode d'administration, et assurent une libération contrôlée et reproductible de ce dernier.

L'un des objets de l'invention était de mettre au point une composition pharmaceutique solide, à libération contrôlée, contenant un simple mélange de Périndopril ou de ses sels pharmaceutiquement acceptables, et de polymère(s) ayant des propriétés plastiques, le(s)dit(s) polymère(s) étant constitué(s) par le groupe des polyméthacrylates, sans ajout de plastifiant, et/ou de retardant, et sans utilisation de solvant.

D'une façon surprenante, les compositions pharmaceutiques solides de la Demanderesse, de part leur constitution spécifique, peuvent aussi bien être soumises à la technique de l'extrusion, de la co-extrusion qu'à celle de l'injection ou de la co-injection. La mise en oeuvre de ces techniques permet d'aboutir à l'obtention de matrices sous des formes de taille et de géométrie appropriées aux diverses voies d'administration telles que notamment la voie orale, buccale, sublinguale, oculaire, vaginale, rectale, et parentérale. Cet avantage des compositions pharmaceutiques de ladite invention permet d'envisager la fabrication, à partir de la même matière première, de la formulation galénique la mieux adaptée à la fois au Périndopril et à ses sels pharmaceutiquement acceptables incorporé dans ladite composition, et à sa meilleure voie d'administration.

Enfin, un des objets de l'invention était d'obtenir une composition pharmaceutique solide où il était possible de moduler simplement la libération du Périndopril, par simple adaptation des quantités de celui-ci et de polymères plastiques utilisés.

Plus spécifiquement, la présente invention concerne une composition pharmaceutique solide thermoformée à libération contrôlée, administrable notamment par voie orale, contenant un mélange thermoformable, de Périndopril et de ses sels pharmaceutiquement acceptables et d'un ou plusieurs polymères choisis parmi le groupe des polyméthacrylates, la libération contrôlée du Périndopril étant uniquement assurée par la nature chimique, la quantité du ou des polyméthacrylates utilisés, et la technique mise en oeuvre pour la fabrication de ladite composition.

Dans les compositions pharmaceutiques selon l'invention, le Périndopril se trouve préférentiellement sous la forme de sel de *tert*-butylamine.

Par composition pharmaceutique à libération contrôlée, on comprend une libération du Périndopril sur une durée de quelques minutes correspondant à une libération immédiate, à une durée de plus de 20 heures correspondant à une libération prolongée, ladite libération pouvant s'effectuer de façon décalée dans le temps après absorption de la composition. Dans le cas de composition pharmaceutique à libération décalée, le temps de latence correspondant au temps entre l'absorption de ladite composition et la libération du principe actif, peut être d'une durée de 30 minutes à 8 heures, la libération du principe actif pouvant ensuite être une libération immédiate ou une libération prolongée telle que définis précédemment. Dans le cadre de l'invention, il est également possible d'obtenir des compositions pharmaceutiques présentant une combinaison des profils de libération telle qu'une libération immédiate d'une partie du principe actif suivie d'une ou plusieurs libérations décalées.

Par polyméthacrylate, on entend un copolymère d'acide méthacrylique correspondant à un copolymère totalement polymérisé d'acide méthacrylique et d'ester acrylique ou méthacrylique. Ces polyméthacrylates sont couramment appelés Eudragit®, et peuvent se présenter sous la forme d'une poudre ou de granulés.

Par mélange thermoformable, on entend un mélange apte à subir une transformation par l'effet combiné de la chaleur et des forces de cisaillement d'une vis sans fin comme les techniques de l'extrusion, de la co-extrusion, de l'injection, et de la co-injection.

Parmi les différents Eudragit® commercialisés, ceux utilisés préférentiellement dans le cadre de l'invention sont les Eudragit® RL et RS qui se réfèrent à des copolymères de méthacrylate ammonium consistant en des copolymères totalement polymérisés d'acide acrylique et d'ester d'acide méthacrylique contenant une faible quantité de groupes ammonium quaternaire.

Ces Eudragit® répondent à la formule générale (I) : 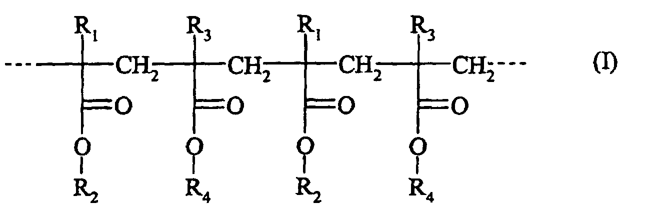 dans laquelle :
R₁ représente un atome d'hydrogène ou un groupement méthyle,
R₂ représente un groupement méthyle ou éthyle,
R₃ représente un groupement méthyle,
et R₄ représente un groupement

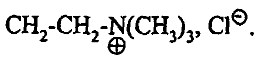

D'une façon particulièrement avantageuse, les Eudragit® utilisés au sein du mélange thermoformable de l'invention, sont les Eudragit® RLPO et/ou RSPO correspondant à des poly(éthyle acrylate, méthyle méthacrylate, chlorure de triméthylaminoéthyle méthacrylate) dans des proportions relatives respectives de 1:2:0,2 et 1:2:0,1.

Selon une autre variante avantageuse de l'invention, le mélange thermoformable de l'invention peut contenir de l'Eudragit® de type E. Ce polymère correspond à un poly(butylméthacrylate, (2-diméthylaminoéthyl)méthacrylate, méthylméthacrylate) dans des proportions relatives 1:2:1. L'Eudragit® de type E peut être utilisé comme seul polymère polyméthacrylate, au sein du mélange thermoformable, ou en association avec de l'Eudragit® RLPO et/ou RSPO, ou avec un ou plusieurs des Eudragit® cités précédemment.

Parmi les Eudragit® de type E, on peut citer notamment l'Eudragit® E100 dont la particularité est d'être soluble à des pH inférieurs à 5, permettant une libération rapide du principe actif au niveau gastrique. De ce fait, l'utilisation d'Eudragit® de type E, et plus particulièrement de type E100, est particulièrement bien adaptée à l'obtention de compositions pharmaceutiques solides à libération immédiate, administrables par voie orale.

Selon une troisième variante de l'invention, le mélange thermoformable de l'invention peut contenir de l'Eudragit® de type L100, L100-55 et/ou S100. L'Eudragit® L100 correspond à un poly(méthacrylique acide, méthylméthacrylate) dans des proportions relatives 1:1. L'Eudragit® L100-55 correspond à un poly(méthacrylique acide, éthylacrylate) dans des proportions relatives 1:1. L'Eudragit® S100 correspond à un poly(méthacrylique acide, méthylméthacrylate) dans des proportions relatives 1:2. Ces catégories d'Eudragit® peuvent être utilisées comme seul polymère polyméthacrylate au sein du mélange thermofonmable ou en association avec un ou plusieurs des autres types d'Eudragit® cités précédemment. Ces polyméthacrylates sont solubles à des pH supérieurs à 5,5, permettant ainsi une libération de principe actif au niveau intestinal et/ou colonique. L'utilisation de cesdits Eudragit® est particulièrement intéressante pour l'obtention de compositions pharmaceutiques solides gastrorésistantes, à libération contrôlée.

Les compositions pharmaceutiques ainsi obtenues dans le cadre de l'invention permettent d'obtenir, de façon inattendue, une libération contrôlée du Périndopril sur une durée de quelques minutes à plus de 20 heures, celle-ci pouvant être linéaire, la constitution de la matrice, et la technique mise en oeuvre.

Ainsi, les compositions pharmaceutiques de l'invention sont obtenues par mélange du Périndopril ou de ses sels pharmaceutiquement acceptables et d'un ou plusieurs polymères polyméthacrylates, abaissement de la viscosité de ce mélange sous l'effet de la chaleur et des forces de cisaillement d'une vis sans fin à l'intérieur d'un fourreau, puis traitement de ce mélange fondu selon l'une des voies suivantes :
- soit expulsion de l'extrudeuse à travers un orifice calibré, de taille et de forme variable, le matériau obtenu étant ensuite découpé selon la taille finale souhaitée de la matrice. Ceci constitue la technique de l'extrusion simple,
- soit la première extrudeuse contenant ledit mélange, de viscosité réduite, décrit précédemment est associée à une seconde extrudeuse contenant un mélange comportant :
   * soit uniquement un ou plusieurs polyméthacrylate(s) pour le contrôle de la libération du principe actif à partir du compartiment central,
   * soit un ou plusieurs polyméthacrylate(s) en mélange avec un ou plusieurs principe(s) actif(s), identique(s) ou différent(s) de celui (ou ceux) contenu(s) dans le compartiment central,
   chaque extrudeuse fonctionnant en continu et alimentant le même orifice.
   Cet orifice permet le passage du mélange provenant de la première extrudeuse et assure la formation de la couche interne de la matrice finale, ainsi que le passage du mélange provenant de la seconde extrudeuse et assurent la formation de la couche externe de la matrice finale. L'extrudat ainsi obtenu est ensuite découpé selon la taille finale souhaitée, et peut éventuellement subir un moulage. Les extrémités de l'extrudat peuvent éventuellement être fermées par une technologie appropriée. Ceci constitue la technique de co-extrusion,
- soit injection sous pression, au sein d'une presse, dans des moules de forme et de volume parfaitement définis selon les caractéristiques géométriques souhaitées pour la matrice. Ceci constitue la technique de l'injection,
- soit la presse est équipée de plusieurs unités d'injection permettant l'injection dans un même moule, de façon séquentielle ou simultanées, d'au moins deux mélanges, identiques ou différents. La première unité d'injection injecte ledit mélange, décrit précédemment, celui-ci constituant la partie centrale, ou coeur, de la matrice. La deuxième unité d'injection injecte à la périphérie de la partie centrale, une couche externe d'un mélange comportant :
   * soit uniquement un ou plusieurs polyméthacrylate(s) pour le contrôle de la libération du Périndopril ou de ses sels d'addition,
   * soit un ou plusieurs polyméthacrylate(s) en mélange avec le Périndopril ou un de ses sels d'addition, identique(s) ou différent(s), de celui (ou ceux) contenu(s) dans la partie centrale.
Ceci constitue la technique de co-injection qui regroupe à la fois les techniques d'injection multimatières et d'injection "sandwich".

Selon la technique employée, il est donc possible d'obtenir dans le cadre de la présente invention des compositions pharmaceutiques solides, administrables notamment par voie orale, buccale, sublinguale, oculaire, rectale, vaginale ou parentérale, à libération contrôlée, de taille et de géométrie variées, monocouche ou multicouche, adaptées au mieux selon les profils de libération les plus adéquates pour le Périndopril.

Ces compositions pharmaceutiques peuvent être utilisées directement, sans autre opération technique de transformation, hormis leur conditionnement. Si on le souhaite, lesdites compositions pharmaceutiques peuvent toutefois subir une transformation par broyage ou par granulation pour une mise en gélule ou pour être comprimées, ou être soumise à un enrobage.

Les compositions pharmaceutiques de l'invention peuvent aussi contenir éventuellement des excipients, pharmacologiquement acceptables, choisis par exemple parmi le groupe des antioxydants, des aromatisants, des colorants, des conservateurs, des édulcorants, et des antiadhérents.

La température de thermoformage est comprise entre 60°C et 150°C. D'une façon préférentielle, la température est comprise entre 80 et 130°C.

Les exemples suivants illustrent l'invention mais ne la limitent en aucune façon.

### EXEMPLE 1 : Extrusion

Les compositions de l'exemple 1 sont obtenues par la technique de l'extrusion. Elles sont toutes dosées à 4 mg de Périndopril, sel de *tert*-butylamine.
Les compositions sont constituées d'un mélange comportant 2 % de Périndopril, sel de *tert*-butylamine et 98 % de polyméthacrylate.
Cet exemple montre l'influence de la nature des polytnéthacrylates utilisés sur la cinétique de dissolution in vitro du principe actif.
La température d'extrusion pour les lots avec Eudragit® RLPO et RSPO est comprise entre 105°C et 110°C et est de 95°C pour le lot avec l'Eudragit® E100. L'extrusion est réalisée avec une filière de 2 mm de diamètre et la vitesse de la vis est de 10 tours/min.

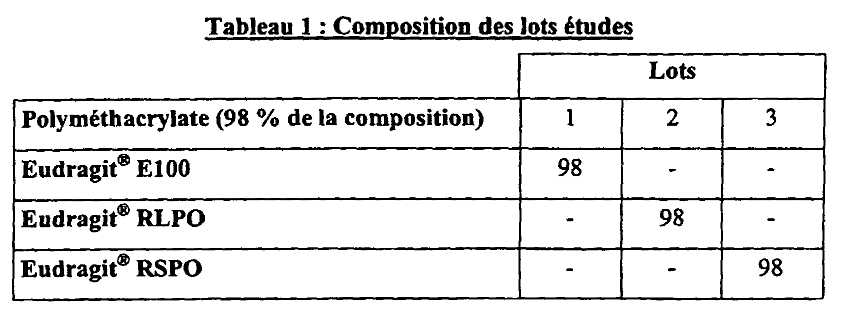

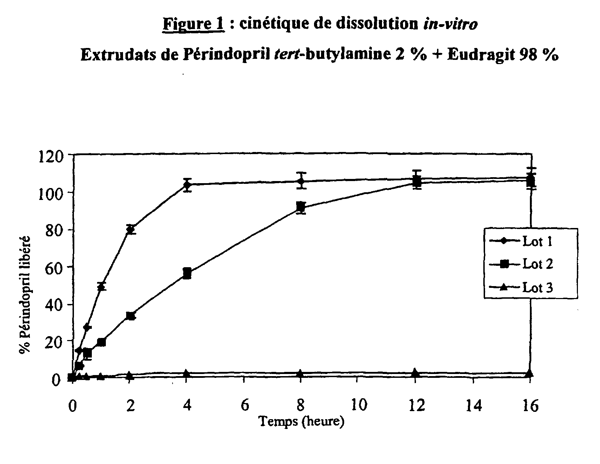

Les cinétiques de dissolution in vitro ont été mesurées par dosage en chromatographie liquide haute performance (HPLC) d'extrudats contenant environ 4 mg de Périndopril, sel de *tert*-butylamine dans un milieu de dissolution tamponné de pH2.
Les cinétiques de dissolution sont présentées en annexe dans la figure 1.

Les résultats montrent que selon le type d'Eudragit® utilisé, la cinétique de libération de Périndopril, sel de *tert*-butylamine peut être modulée entre par exemple 100 % de produit libéré instantanément et 100 % de produit libéré en 12 heures.

D'autre part, et de manière surprenante, il a été montré que le Périndopril, sel de *tert* butylamine, habituellement instable à la température ou avec certains excipients acides n'était pas ou peu dégradé lors de cette extrusion à chaud qui s'effectue à des températures comprises entre 95°C et 110°C (moins de 3 % de dégradation).

Enfin, une étude de stabilité sur 6 mois dans des conditions de température et d'humidité relative variable a permis de montrer une excellente stabilité du Périndopril, sel de *tert*-butylamine dans un conditionnement de type blister aluminium/PVC.

### EXEMPLE 2 : Co-extrusion

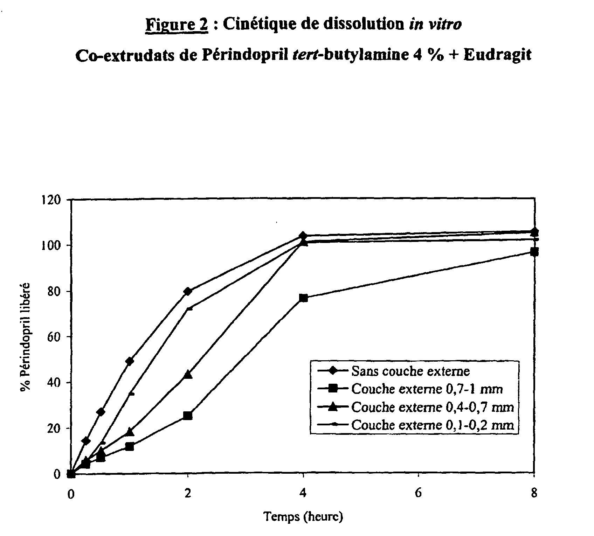

Les compositions de cet exemple ont été obtenues par la technique de co-extrusion. Ces compositions sont dosées à 4 mg de Périndopril, sel de *tert*-butylamine.
La couche interne est constituée d'un mélange contenant 4 % de Périndopril, sel de *tert*-butylamine et 96 % d'Eudragit® E100. La couche externe est constituée à 100 % d'Eudragit® RLPO.
La présence d'une couche externe de polyméthacrylate RLPO permet de ralentir la cinétique de libération du principe actif depuis la couche interne.
Les températures de co-extrusion sont de 85°C pour la couche interne et comprises entre 80 et 105°C pour la couche externe.
Les cinétiques de dissolution in vitro ont été mesurées par HPLC par dosage d'extrudats contenant 4 mg de sel de *tert*-butylamine de Périndopril.
Les cinétiques de dissolution sont présentées en annexe dans la figure 2.

Les résultats indiquent que l'ajout d'une couche externe permet d'une part de linéariser la cinétique de libération du Périndopril et/ou de la ralentir. Plus l'épaisseur de la couche externe est importante, plus la cinétique de libération est ralentie.

## Revendications

1. Composition pharmaceutique solide thermoformée pour la libération contrôlée de Périndopril ou de ses sels pharmaceutiquement acceptables, **caractérisée en ce qu'**elle comporte un mélange thermoformable de Périndopril ou d'un de ses sels pharmaceutiquement acceptables et d'un ou plusieurs polymères choisis parmi le groupe des polyméthacrylates, la libération du Périndopril étant uniquement contrôlée par la nature du ou des polyméthacrylates utilisés, leur quantité relative par rapport au Périndopril, et par la technique mise en oeuvre pour la fabrication de ladite composition.

2. Composition pharmaceutique solide à libération contrôlée selon la revendication 1 **caractérisée en ce que** le ou les polyméthacrylate(s) utilisé(s) dans le mélange thermoformable appartienne(nt) à la famille des Eudragit® RL et/ou RS.

3. Composition pharmaceutique solide à libération contrôlée selon l'une quelconque des revendications 1 et 2 **caractérisée en ce que** le ou les polyméthacrylate(s) utlisé(s) dans le mélange thermoformable est (sont) de l'Eudragit® RLPO et/ou de l'Eudragit® RSPO.

4. Composition pharmaceutique solide à libération contrôlée selon l'une quelconque des revendications 1 à 3 **caractérisée en ce que** le mélange thermoformable comporte de l'Eudragit® de type E, seul ou en association avec un ou plusieurs des Eudragit® cités précédemment.

5. Composition pharmaceutique solide à libération contrôlée selon l'une quelconque des revendications 1 à 4, **caractérisée en ce que** le mélange thermoformable comporte de l'Eudragit® de type L100, L100-55 et/ou S100, seul(s) ou en association avec un ou plusieurs des Eudragit® cités précédemment.

6. Composition pharmaceutique solide à libération contrôlée selon la revendication 1, **caractérisée en ce que** ladite composition est administrable selon l'une des voies choisies parmi orale, buccale, sublinguale, oculaire, vaginale, rectale et parentérale.

7. Composition pharmaceutique solide à libération contrôlée selon l'une quelconque des revendications 1 à 6, **caractérisée en ce que** ladite composition est administrable par voie orale.

8. Composition pharmaceutique solide à libération contrôlée selon la revendication 1 **caractérisée en ce que** la température de thermoformage du mélange est comprise entre 60°C et 150°C.

9. Composition pharmaceutique solide à libération contrôlée selon l'une quelconque des revendications 1 et 8, **caractérisée en ce que** la température de thermoformage du mélange est comprise entre 80°C et 130°C.

10. Composition pharmaceutique solide à libération contrôlée selon la revendication 1, **caractérisée en ce que** le mélange est thermoformé selon la technique de l'extrusion.

11. Composition pharmaceutique solide à libération contrôlée selon la revendication 1, **caractérisée en ce que** le mélange est thermoformé selon la technique de l'injection.

12. Composition pharmaceutique solide à libération contrôlée selon la revendication 1, **caractérisée en ce que** le mélange est thermoformé selon la technique de la co-extrusion, la couche interne de ladite composition étant alors constituée par ledit mélange et la couche externe de ladite composition étant constituée par un ou plusieurs polyméthacrylate(s), soit par un ou plusieurs polyméthacrylate(s) en mélange avec le Périndopril ou l'un de ses sels pharmaceutiquement acceptables.

13. Composition pharmaceutique solide à libération contrôlée selon la revendication 1, **caractérisée en ce que** le mélange est thermoformé selon la technique de la co-injection, la partie centrale de ladite composition étant alors constituée par ledit mélange et la couche externe de ladite composition étant constituée soit par un ou plusieurs polyméthacrylate(s), soit par un ou plusieurs polyméthacrylate(s) en mélange avec un le Périndopril ou l'un de ses sels pharmaceutiquement acceptables.

14. Composition pharmaceutique solide à libération contrôlée selon la revendication 1, **caractérisée en ce qu'**elle comporte éventuellement un ou plusieurs excipients, pharmacologiquement acceptables, choisis parmi les antioxydants, les aromatisants, les colorants, les conservateurs, les édulcorants et les antiadhérents.

15. Composition pharmaceutique solide selon la revendication 1 **caractérisée en ce que** le Périndopril est sous la forme de sel de *tert*-butylamine.

## Patentansprüche

1. Feste, warmgeformte pharmazeutische Zubereitung für die gesteuerte Freisetzung von Perindopril oder seiner pharmazeutisch annehmbaren Salze, **dadurch gekennzeichnet, daß** sie eine warmformbare Mischung aus Perindopril oder eines seiner pharmazeutisch annehmbaren Salze und ein oder mehrere Polymere ausgewählt aus der Gruppe der Polymethacrylate umfaßt, wobei die Freisetzung von Perindopril ausschließlich über die Art des oder der verwendeten Polymethacrylat(e), deren relative Menge bezogen auf Perindopril und die für die Herstellung der Zubereitung angewandte Technik gesteuert wird.

2. Feste pharmazeutische Zubereitung zur gesteuerten Wirkstofffreisetzung nach Anspruch 1, **dadurch gekennzeichnet, daß** das oder die verwendete(n) Polymethacrylat(e) in der warmformbaren Mischung der Familie der Produkte Eudragit® RL und/oder RS angehört (angehören).

3. Feste pharmazeutische Zubereitung zur gesteuerten Wirkstofffreisetzung nach einem der Ansprüche 1 und 2, **dadurch gekennzeichnet, daß** das oder die in der warmformbaren Mischung verwendete(n) Polymethacrylat(e) Eudragit® RLPO und/oder Eudragit® RSPO ist (sind).

4. Feste pharmazeutische Zubereitung zur gesteuerten Wirkstofffreisetzung nach einem der Ansprüche 1 bis 3, **dadurch gekennzeichnet, daß** die warmformbare Mischung Eudragit® Typ E allein oder in Kombination mit einem oder mehreren der oben genannten Eudragit®-Produkte umfaßt.

5. Feste pharmazeutische Zubereitung zur gesteuerten Wirkstofffreisetzung nach einem der Ansprüche 1 bis 4, **dadurch gekennzeichnet, daß** die warmformbare Mischung Eudragit® Typ L100, L100-55 und/oder S100 allein oder in Kombination mit einem oder mehreren der oben genannten Eudragit®-Produkte umfaßt.

6. Feste pharmazeutische Zubereitung zur gesteuerten Wirkstofffreisetzung nach Anspruch 1, **dadurch gekennzeichnet, daß** die Zubereitung nach einem Verabreichungsweg verabreicht werden kann, welcher aus der oralen, bukkalen, sublingualen, okularen, vaginalen, rektalen und parenteralen Verabreichung ausgewählt ist.

7. Feste pharmazeutische Zubereitung zur gesteuerten Wirkstofffreisetzung nach einem der Ansprüche 1 bis 6, **dadurch gekennzeichnet, daß** die Zubereitung auf oralem Wege verabreicht werden kann.

8. Feste pharmazeutische Zubereitung zur gesteuerten Wirkstofffreisetzung nach Anspruch 1, **dadurch gekennzeichnet, daß** die Temperatur bei dem Warmformen der Mischung zwischen 60°C und 150°C liegt.

9. Feste pharmazeutische Zubereitung zur gesteuerten Wirkstofffreisetzung nach einem der Ansprüche 1 bis 8, **dadurch gekennzeichnet, daß** die Temperatur bei dem Warmformen der Mischung zwischen 80°C und 130°C liegt.

10. Feste pharmazeutische Zubereitung zur gesteuerten Wirkstofffreisetzung nach Anspruch 1, **dadurch gekennzeichnet, daß** die Mischung nach der Extrusionstechnik warmgeformt ist.

11. Feste pharmazeutische Zubereitung zur gesteuerten Wirkstofffreisetzung nach Anspruch 1, **dadurch gekennzeichnet, daß** die Mischung nach der Spritz-Technik warmgeformt ist.

12. Feste pharmazeutische Zubereitung zur gesteuerten Wirkstofffreisetzung nach Anspruch 1, **dadurch gekennzeichnet, daß** die Mischung mit der Co-Extrusionstechnik warmgeformt ist, wobei die innere Schicht der Zubereitung durch die genannte Mischung gebildet wird und die äußere Schicht der genannten Zubereitung durch ein oder mehrere Polymethacrylat(e) oder durch ein oder mehrere Polymethacrylat(e) in Mischung mit Perindopril oder einem seiner pharmazeutisch annehmbaren Salze gebildet wird.

13. Feste pharmazeutische Zubereitung zur gesteuerten Wirkstofffreisetzung nach Anspruch 1, **dadurch gekennzeichnet, daß** die Mischung mit der Co-Spitztechnik warmgeformt ist, wobei der zentrale Bereich der Zubereitung durch die genannte Mischung gebildet wird und die äußere Schicht der genannten Zubereitung entweder durch ein oder mehrere Polymethacrylat(e) oder ein oder mehrere Polymethacrylat(e) in Mischung mit Perindopril oder einem seiner pharmazeutisch annehmbaren Salze gebildet wird.

14. Feste pharmazeutische Zubereitung zur gesteuerten Wirkstofffreisetzung nach Anspruch 1, **dadurch gekennzeichnet, daß** sie gegebenenfalls ein oder mehrere pharmazeutisch annehmbare Hilfsstoffe umfaßt, ausgewählt aus Antioxidantien, Aromatisierungsmitteln, Farbstoffen, Konservierungsmitteln, Süßungsmitteln und Haftverhinderungsmitteln.

15. Feste pharmazeutische Zubereitung zur gesteuerten Wirkstofffreisetzung nach Anspruch 1, **dadurch gekennzeichnet, daß** das Perindopril in Form des tert.-Butylaminsalzes vorliegt.

## Claims

1. Solid thermoformable pharmaceutical composition for the controlled release of perindropil or a pharmaceutically acceptable salt thereof, **characterised in that** it comprises a thermoformable mixture of perindopril or a pharmaceutically acceptable salt thereof and of one or more polymers selected from the group of the polymethacrylates, the release of the perindopril being controlled solely by the nature of the polymethacrylate(s) used, by the amount thereof relative to the perindopril and by the technique employed in the manufacture of the said composition.

2. Solid controlled-release pharmaceutical composition according to claim 1, **characterised in that** the polymethacrylate(s) used in the thermoformable mixture belong(s) to the family of the Eudragit® products RL and/or RS.

3. Solid controlled-release pharmaceutical composition according to either claim 1 or claim 2, **characterised in that** the polymethacrylate(s) used in the thermoformable mixture is/are Eudragit® RLPO and/or Eudragit® RSPO.

4. Solid controlled-release pharmaceutical composition according to any one of claims 1 to 3, **characterised in that** the thermoformable mixture comprises Eudragit® of type E, alone or in association with one or more of the Eudragit® products mentioned hereinbefore.

5. Solid controlled-release pharmaceutical composition according to any one of claims 1 to 4, **characterised in that** the thermoformable mixture comprises Eudragit® of type L100, L100-55 and/or S100, alone or in association with one or more of the Eudragit® products mentioned hereinbefore.

6. Solid controlled-release pharmaceutical composition according to claim 1, **characterised in that** the said composition is administrable by one of the routes selected from the oral, buccal, sublingual, ocular, vaginal, rectal and parenteral routes.

7. Solid controlled-release pharmaceutical composition according to any one of claims 1 to 6, **characterised in that** the said composition is administrable by the oral route.

8. Solid controlled-release pharmaceutical composition according to claim 1, **characterised in that** the temperature of thermoforming of the mixture is from 60°C to 150°C.

9. Solid controlled-release pharmaceutical composition according to either claim 1 or claim 8, **characterised in that** the temperature of thermoforming of the mixture is from 80°C to 130°C.

10. Solid controlled-release pharmaceutical composition according to claim 1, **characterised in that** the mixture is thermoformed according to the technique of extrusion.

11. Solid controlled-release pharmaceutical composition according to claim 1, **characterised in that** the mixture is thermoformed according to the technique of injection.

12. Solid controlled-release pharmaceutical composition according to claim 1, **characterised in that** the mixture is thermoformed according to the technique of co-extrusion, the inner layer of the said composition in this case being composed of the said mixture and the outer layer of the said composition being composed either of one or more polymethacrylate(s) or of one or more polymethacrylate(s) in admixture with perindopril or a pharmaceutically acceptable salt thereof.

13. Solid controlled-release pharmaceutical composition according to claim 1, **characterised in that** the mixture is thermoformed according to the technique of co-injection, the central portion of the said composition in this case being composed of the said mixture and the outer layer of the said composition being composed either of one or more polymethacrylate(s) or of one or more polymethacrylate(s) in admixture with perindopril or a pharmaceutically acceptable salt thereof.

14. Solid controlled-release pharmaceutical composition according to claim 1, **characterised in that** it optionally contains one or more pharmacologically acceptable excipients selected from anti-oxidants, flavourings, colourings, preservatives, sweeteners and anti-adherents.

15. Solid pharmaceutical composition according to claim 1, **characterised in that** the perindopril is in the form of the *tert*-butylamine salt.
